# EUROPEAN PATENT APPLICATION

(11) **EP 4 529 912 A1**
(43) Date of publication of application: **02.04.2025**
(21) Application number: 23306649.7
(22) Date of filing: 29.09.2023
(51) Int. Cl.: A61J 1/20, A61J 1/14, A61M 39/18

(54) **CONNECTION SYSTEM FOR OPENING A CONNECTION, PROCESS FOR MANUFACTURING SUCH A SYSTEM AND METHOD FOR OPENING A CONNECTION USING SUCH A SYSTEM**

(71) Applicant: NEMERA LA VERPILLIERE, 38290 La Verpillière (FR)
(72) Inventor: GRENOT, Gaëtan, 74210 Faverges (FR); ROUX, Julien, 38100 Grenoble (FR)
(74) Representative: Lavoix

(57) **Abstract**

This connection system (100) for opening a connection between a first internal volume (V4) of a first element (4) and a second internal volume (V6) of a second element (6). The first internal volume is closed by a first seal membrane (134) and the second internal volume is closed by a second seal membrane (154). The connection occurs via a relative translational movement (A1, A2) between the first and second elements (4, 6) along a main axis (A100) of the connection system. This relative translational movement (A1, A2) induces a displacement of at least a first portion (134A) of the first membrane (134) with respect to the first element (4) and a displacement of at least a first portion (154A) of the second membrane (154) with respect to the second element (6).

## Description

### TECHNICAL FIELD OF THE INVENTION

The present invention concerns a connection system for opening a connection between two internal volumes. The present invention also concerns a process for manufacturing such a connection system and a method for opening the connection using such a connection system.

The invention allows establishing a fluid connection between two internal volumes of the two elements, while a sterility is maintained in both volumes before the connection. Establishing a connection allows further handling of a fluid or fluids enclosed in one or two volumes. For example, a fluid container may be connected with a system for fluid transfer or two fluids may be mixed with each other.

### BACKGROUND OF THE INVENTION

In many applications, in particular in medicine, there is a need for an efficient method for establishing the connection between two internal volumes of two different elements, among which at least one comprises a fluid, and which have been kept sterile before opening the connection. For providing the sterility, various type of seal members are used, wherein removing of the seal members allows to open the connection. The seal members need to be reliably attached to the two element, in order to reduce the risk of their accidental removing and the risk of loosing sterility. On the other hand, the intended process of removing the seal members should be simple and designed so that it can be performed the elderly or disabled people. For this purpose, it should not require too many steps or use of additional devices other than the two elements which are to be connected.

In one exemplary application the present invention may be used for providing the connection between a drug container and a drug delivery device.

Whatever the embodiment envisaged, the drug delivery device according to the invention can, for example, be used to administer a drug in liquid form to a patient in order to treat a wide variety of pathologies such as, for example, autoimmune diseases or chronic diseases such as diabetes or associated disorders, cancers, hormonal insufficiency, macular degeneration, inflammation, atherosclerosis, rheumatoid arthritis, coronary syndromes, thromboembolic diseases, etc.

Thus, the term "drug" designates any liquid formulation capable of being administered continuously by means, for example, of a hollow needle or a cannula. The formulation can be, for example, a solution, a gel or a fine suspension containing one or more active ingredients. These active ingredients may be, in particular, peptides (for example, insulin or GLP-1, their derivatives or analogues, amylin or its derivatives or analogues, gastric inhibitory peptides or their analogues), proteins, glycoproteins or hormones (for example hormones from pituitary gland or hypothalamus, such as gonadotropin or gonadotropin releasing hormone, desmopressin, gonadorelin, triptorelin, terlipressin, leuprorelin, buserelin, goserelin, nafarelin, lutropin, menotropin, follicle - stimulating hormone and its analogues, follitropin, parathyroid hormone, teriparatide, abaloparatide or other analogues of parathyroid hormone, calcitonin, growth hormone, somatropin, etc., active ingredients derived from hormones or nucleotides (e.g. DNA, RNA, oligonucleotides), enzymes, polysaccharides (e.g. glycosaminoglycans, hyaluronic acids, heparins, low molecular weight heparins and their derivatives, or sulfated or forms poly-sulfated of these polysaccharides), vaccines, antibodies and their analogues (for example denosumab or panitumumab) or nutritional substances. The formulations may contain these active ingredients in the form of any pharmaceutically acceptable salt or solvate as well as any necessary and appropriate excipients.

Drug containers used for drug delivery may have the form of a barrel, preferably made of a glass or a plastic material, which is closed at one end by a septum, preferably disposed on e.g. a narrow end portion of the drug container, preferably providing pierceable sterile barrier. On the other end, the drug containers may be sealed by a movable stopper, preferably made from an elastomeric material. When the content of the drug container is to be used, the septum needs to be pierced to connect an internal volume of the drug container with the drug delivery device, i.e. to open a connection or a fluid path between the internal volume of the container and drug delivery device. In order to guarantee a long-lasting sterility of such a container, the septum may be covered by a layer of material preventing contamination from the outside. Sterility should also be provided for a needle used for piercing the septum

In another exemplary application the present invention may be used in a system for mixing two fluids.

In yet another exemplary application the present invention may be used in a system for reconstitution. There are many examples, in particular in a field of medicine, in which the desired fluid may be obtained by dissolving of the substance in a solid state in a fluid dissolvent. It is particularly useful.

WO-A-2017/219154 discloses a drug delivery device in which a fluid path between an internal volume of a drug container and a drug delivery system is opened by piercing a septum with a spike. The fluid path is provided within the device and both the fluid path and the drug container must be sterilized. A peel-off foil, attached to a housing of the device, keeps the internal volume of the device sterile and must be removed just before use of the device. This requires keeping both the drug container and the fluid path in a sterile environment during assembly and does not allow, for example, changing the drug container and using more than one drug container with the same drug delivery system since, each time the drug container is assembled in the device, this must be done in a sterile environment, which is found only in some facilities.

US-A-2019/0374707 discloses a drug delivery device with a system for opening a fluid path between a cartridge and a seal member, which is further connected to a flexible tubing. In order to provide sterility of the fluid path, two removable membranes are used for covering exterior end surfaces of the cartridge and the seal member. The removable membranes are in a folded configuration, which facilitates easier removal by pulling the membranes substantially linearly. The membranes are pulled by a removal member and their specific shape reduces the force needed for the removal, as only a part of the adhesive force needs to be overcome at once.

WO-A-2011/015659 discloses a medication delivery device comprising a base part, which can be combined with a detachable drug reservoir or delivery part. A fluid path is provided by means of a connection part provided with a connection needle. Both the connection part and the drug reservoir are provided with a bubble shaped self-closing membrane allowing separating and rejoining them without contamination. Shaping the self-closing membranes as bubbles is a sophisticated and complicated solution, which negatively impacts the cost of the device. Moreover, the bubbles need to be pierced by the needle. There is still a risk of contamination related with such contact.

The prior art solutions do not provide a reliable method for automatic connection and opening of a connection, which would be simple and which would keep sterility of both the first and second elements, even when said elements are placed in a non-sterile environment. In particular there is no solution in which opening the connection would require only a relative movement between the elements which are to be connected and would not require additional piercing of any of the sealing elements.

### SUMMARY OF THE INVENTION

The present invention aims at solving these problems with a new connection system, which allows opening a connection by relative axial movement between a first element and a second element, without any action other than a relative axial movement to be performed by the user.

To this end, according to a first aspect, the invention concerns a connection system for opening a connection between a first internal volume of a first element, the first volume being closed by a first seal membrane, and a second internal volume of a second element, the second volume being closed by a second sealmembrane, wherein the connection occurs via a relative translational movement between the first and second elements along a main axis of the connection system. According to the invention, the relative translational movement between the first and second elements induces a displacement of at least a first portion of the first membrane with respect to the first element and a displacement of at least a first portion of the second membrane with respect to the second element.

Thanks to the invention, the first and second seal membranes keep sterility of the first and second internal volumes. This sterility is maintained as long as the first and second seal membranes remain attached to the first and second elements. These seal membranes may be easily peeled-off, without a need of any additional action from the user, as a consequence of the relative translational movement between the first and second elements, along the main axis.

Some portions of the seal members may remain attached after opening the connection or may be detached from the first and second element, respectively.

In the meaning of the present invention, an attachment between two parts is any property allowing control of the force needed to separate these two parts from each other, for example the type and the amount of the adhesive used, the surface roughness, the shape of the connected parts, any combination of these properties or any other property known by the skilled person.

In the present specification, a relative translational movement between two elements should be understood as any of the:
- displacement of the first element toward the second element substantially along the main longitudinal axis of a system or
- displacement of the second element toward the first element substantially along the main longitudinal axis of a system or
- displacement of both the first and the second element toward each other substantially along the main longitudinal axis of a system.

According to advantageous aspects of the invention, such a connection system might incorporate one or several of the following features:
- The first element and the second element comprise engagement members, preferably in the form of snap members.
- One of the first element and second element comprises a through hole covered by a perforable element and a seal membrane and the other one of the first element and second element comprises a piercing member configured to pierce the perforable element to open the connection.
- The other one of the first element and the second element, which comprises the piercing member, further comprises a slidable closure member located on a side of the other one of the first element and the second element directed toward the one of the first element and second element, wherein the slidable closure further comprises a through hole aligned with a tip of the piercing member.
- The slidable closure member is equipped with at least one sealing member configured for making a tight connection between the slidable closure member and a body of the other one of the first element and the second element.
- One of the first seal membrane and the second seal membrane is attached to the slidable closure member.
- The first element and the second element comprise protruding reliefs configured to push parts of the seal membranes disposed on the other of the first element and the second element and causing removing of the seal membranes and opening the connection.
- The first seal membrane includes a first portion which is substantially perpendicular to the main axis and attached to the first element, a second portion substantially parallel to the main axis and attached to the first element, and a third portion connecting the first portion to the second portion. The second seal membrane includes a first portion which is substantially perpendicular to the main axis and attached to the second element, a second portion substantially parallel to the main axis and attached to the second element, and a third portion connecting the first portion to the second portion. Before connection, the third portions of the first and second seal membranes extend at least partially in a direction non parallel to the main axis and the first seal membrane and the second seal membrane are configured so that during opening the connection between the first element and the second element, said first portions detach from the first element while said second portions remains attached to the first element and second element.
- The first element and the second element are parts of a drug delivery system.
- The drug delivery system includes a disposable part comprising a drug container and a reusable part comprising a mechanism for delivering the drug, whereas the connection system allows to open a fluid path between the drug container and another element of the disposable part.
- The first element and the second elements are parts of a mixing system or reconstitution system.

According to a second aspect, the invention concerns a manufacturing process for manufacturing the connection system of any of the invention, comprising at least the following steps:
a. providing the first and the second element having the first volume and the second volume, respectively, wherein the first volume and the second volume are open,
b. providing a first seal membrane and a second seal membrane
c. attaching the first seal membrane to the first element and the second seal membrane to the second element causing closing of the first volume and the second volume, respectively,
characterized in that the attachment of the first seal membrane and the attachment of the second seal membrane are configured so that said membranes can be displaced in result of the relative translational movement between the first element and the second element, causing opening of the connection.

According to advantageous aspects of the invention, such a manufacturing process might incorporate one or several of the following features:
The first seal membrane includes a first portion, substantially perpendicular to the main axis and attached to the first element, a second portion, substantially parallel to the main axis and attached to the first element, and a third portion connecting the first portion to the second portion. The second seal membrane includes a first portion which is substantially perpendicular to the main axis and attached to the second element, a second portion substantially parallel to the main axis and attached to the second element, and a third portion connecting the first portion to the second portion. At least the first portion of the first seal membrane and the second portion of the first seal membrane are attached to the first element and at least the first portion of the second seal membrane and the second portion of the second seal membrane are attached to the second element. Attachment of the first seal membrane and the second seal membrane is configured so that during opening the connecting between the first element and the second element, said first portions detach from the first element and the second element before said second portions detach from the first element and second element.
- Attaching the first seal membrane to the first element and the second seal membrane to the second element is made with the use of adhesive, preferably adhesive supported by the first seal membrane and the second seal membrane, or welding.

According to a third aspect, the invention concerns a method for opening a connection between a first element and a second element using a connection system according to the invention, wherein the method includes at least the following steps consisting in :
d. substantially aligning the first element and the second element along a main axis of the connection system;
e. relative translational movement between the first element and the second element, along the main axis,
f. displacement of at least a part of the first seal membrane with respect to the first element and displacement of at least a part of the second seal membrane with respect to the second element,
g. opening the connection between the first internal volume of the first element and the second internal volume of the second element,
and wherein step f. is a result of step e. and step g. is a result of step f.

This method induces the same advantages as the connecting system of the invention.

According to advantageous but non-compulsory aspects of the invention, such a method might incorporate one or several of the following features:
- The first element and the second element comprise protruding reliefs and the step f is a result of pushing the first seal membrane with the protruding relief of the second element and pushing the second seal membrane with the protruding relief of the first element.
- One of the first element and the second element comprises a perforable element and the other one of the first element and second element comprises a piercing member configured to pierce the perforable element after step g., wherein revealing a tip of the piercing member is a result of a relative translational movement between the first and second elements.

### BRIEF DESCRIPTION OF THE DRAWINGS

The invention will be better understood, based on the following description, which is given in correspondence with the appended figures and as an illustrative example, without restricting the object of the invention. In the annexed figures:
[Fig.1] Figure 1 is a schematic longitudinal cut view of a connection system according to the invention, in a first configuration, before connection;
[Fig.2] Figure 2 is a schematic longitudinal cut view of the system of figure 1 in a second configuration, during connection;
[Fig.3] Figure 3 is a schematic longitudinal cut view of the system of figure 1 in a third configuration, after connection; and.
[Fig.4] Figure 4 shows on three inserts A), B) and C) external perspective views of the connecting system, taken from different angles, respectively in the configurations of figures 1, 2 and 3.

### DETAILED DESCRIPTION OF SOME EMBODIMENTS

The connection system 100 represented on the figures is used for connecting a partly represented drug container 4, which forms a first element, and a receiving part 6, which forms a second element. The drug container 4 and the receiving part 6 are parts of a drug delivery system.

The drug delivery system includes a mechanism for expelling the drug outside said system, preferably to the patient tissue. The mechanism may be of other type, provided that it is able to force drug flow outside the system. The energy needed for this purpose may be taken from a resilient element, for example a spring, may be provided manually by the user or may be supplied from an electric motor.

The drug delivery system may be disposable with the drug container 4 included therein, so that the system is discarded after use, usually when the drug container is empty. Alternatively the drug delivery system may include a reusable part, comprising the mechanism for expelling the drug and a disposable part comprising the drug container 4. In this case, the disposable part can be replaced, which significantly extends the drug delivery system lifetime and reduces the amount of material needed for the same number of drug delivery events.

The described example of the connection system 100 is not limited to a particular type of the drug delivery system. The disposable part of the drug delivery system may include the drug container 4, the receiving part 6 and the connection system, all together. In an alternative embodiment the disposable part includes the drug container, while the reusable part includes the receiving parts and the connection system 100 includes some elements which belong to the disposable part and some other elements which belong to the reusable part. In yet other embodiment the drug container 4, the receiving part 6 and the connection system 100 are all parts of the disposable drug delivery system.

The not represented drug delivery device can be of any type suitable for delivering a drug in a liquid form, for example a wearable device such as on-body injector, syringe, infusion system, pen injector or auto-injector.

In the present specification, a front side or the front of an element or a portion of an element of the fluid connection system 100 is oriented toward the other element when these elements are connected or in the process of being connected. A back side or the back of an element is oriented opposite to its front side or front.

V4 denotes the internal volume of the drug container 4 and V6 denotes the internal volume of the receiving part 6.

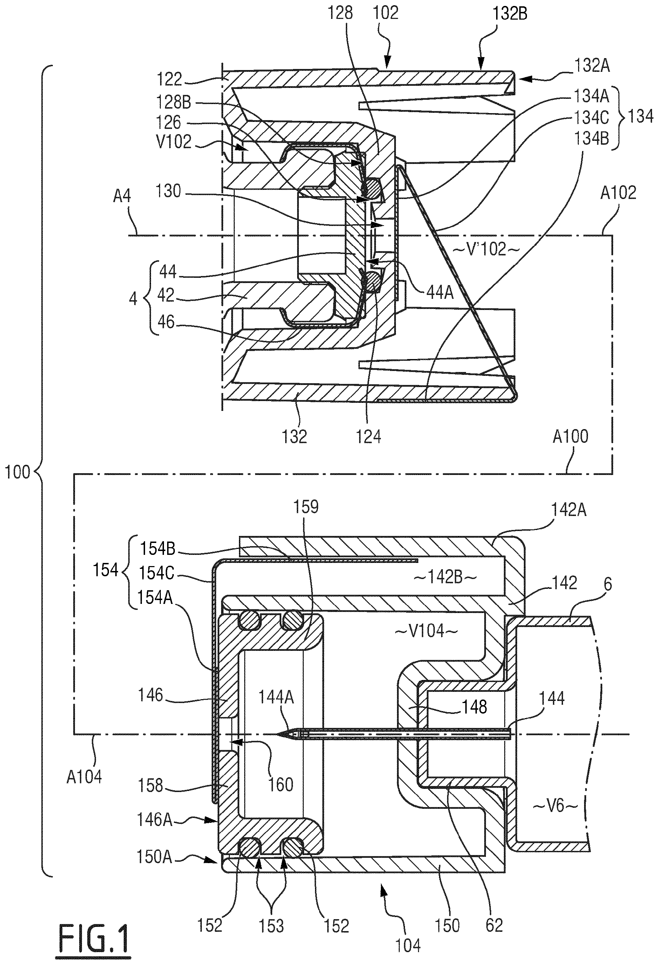

Unless specified, the description of the connection system 100 given here below applies to the first configuration represented on figure 1, before establishing the connection between the drug container 4 and the receiving part 6.

The drug container 4 includes a main body 42 and a septum 44, preferably made of rubber, which has a skirt 442 engaged within an opening 422 defined by a neck 424 of the main body 42. The septum 44 also includes a central area 444 configured to be pierced by a needle, to provide the fluid path and to allow the liquid drug to be transferred from the container inner volume V4. A holding ring 46 is set around the neck 424 and around the septum 44 and holds the septum 44 in position on the main body 42. The holding ring 46 may be provided as a ferrule, for example made of an aluminum or other metal or alloy. A4 denotes a longitudinal main axis of the container 4. Parts 42, 44 and 46 have a substantially circular cross-section centered on this longitudinal axis A4.

The body 42 of the container 4 can be made of glass, or of a synthetic material, for example, a plastic material. Preferably, the body has a form of a barrel closed on the other end by a non-represented stopper made of a flexible and fluid impermeable material, for example rubber.

44A denotes an external surface of the septum 44.

The connection system 100 of the invention includes a cap 102 configured to be mounted on the container 4. More precisely, the cap comprises a body 122 that defines a volume V102 configured to accommodate the neck 424 equipped with the septum 44 and the holding ring 46 of the container 4. The cap 102 is thus configured to be mounted on the container 4, in a position where it covers the central area 444, in particular the external face 44A of the septum 44.

Advantageously, the body 122 is made of an integral piece of synthetic material, preferably a material that can be molded, for example acrylonitrile butadiene styrene, methylmethacrylate acrylonitrile butadiene styrene or MABS, polyamide, polybutylene terephthalate, polycarbonate, polyoxymethylene (acetal), polypropylene or polytetrafluoroethylene.

A102 denotes a longitudinal axis of the cap 102. When the cap 102 is mounted on the container 4, the axes A4 and A102 substantially coincide with each other.

A sealing gasket 124 is mounted in a corresponding groove 126 made in an internal wall 128 of the body 122 and lies against the external surface 44A of the septum 44 and/or the holding ring 46, in order to isolate the external surface 44A of the septum 44 from the ambient air.

A through hole 130 extends through the wall 128 and is located in front of the external surface 44A of the septum 44, the front side of the cap 102 being oriented in a direction parallel to the longitudinal axis A102 and away from the non-represented bottom of the container body 42, when the cap 102 is mounted on the drug container 4.

With this convention, the external surface 44A is the front face of the septum 44.

The groove 126 and the through hole 130 are preferably centered on the longitudinal axis A102. The through hole 130 is then in front of the central area 444.

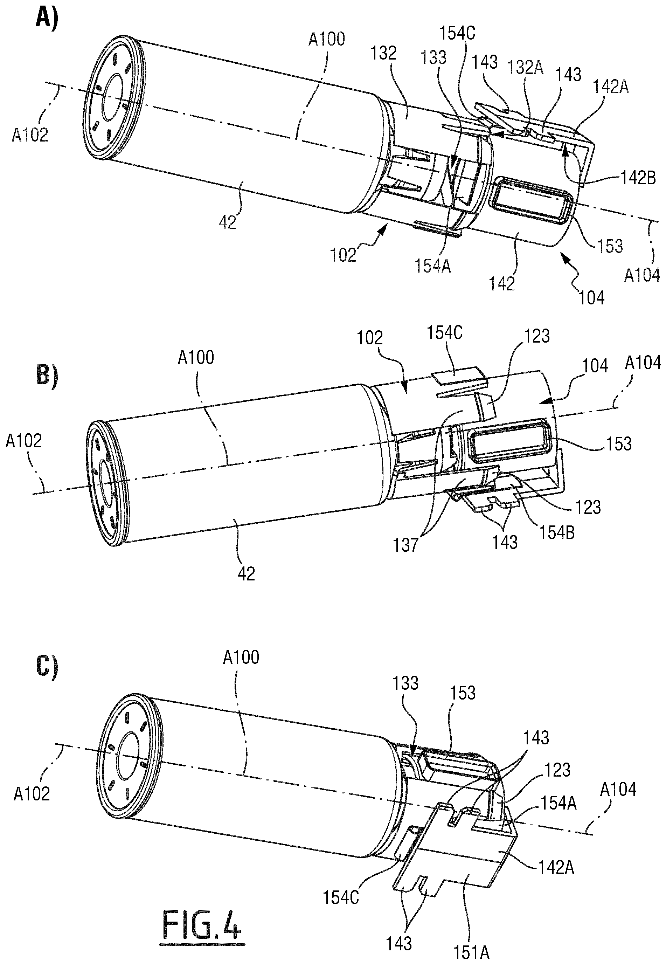

The cap 102, more particularly its body 122, also includes a skirt 132 centered on the longitudinal axis A102. The skirt 132 may include gaps, so that it does not necessarily go all around the longitudinal axis A102. In the example of the figures, and as visible on figure 4, the skirt 132 defines a slot 133.132A denotes a front edge of the skirt 132. This front edge 132A is a protruding relief of the cap 102, oriented toward the front of the cap 102.

128A denotes a front surface of the wall 128, which is oriented toward the front of the cap 102. 128B denotes a rear surface of the wall 128, which is oriented opposite to the front surface, towards the external surface 44A of the septum 44 when the cap 102 is mounted on the container 4. The groove 126 is thus formed on the rear surface 128B of the wall 128. A circular rib 128C, centered on the longitudinal axis A102, protrudes from the front surface 128A of the wall 128 to provide contact surface to be engaged with another part of the connection system 100, as explained here below.

A first seal membrane 134 belongs to the cap 102 and is attached to the body 122 in the way as described below.

In the example, this first membrane 134 can be made of any suitable material impermeable for the liquid and for bacteria, but permeable to air. Particular examples of such materials are non-woven fabrics, such as the non-woven fabric made of high density polyethylene fibers known with a brand name Tyvek^{®}, or the non-woven fabrice made of high density polypropylene fibers known with a brand name PelTek. A porous paper may be used as well as such barrier membrane. Use of a sterile membrane permeable to air is particularly advantageous as it allows to conduct steam sterilization which is commonly used for the drugs which usually cannot be handled with gamma sterilization.

A first portion 134A of the first seal membrane 134 is perpendicular to the longitudinal axis A102 and is attached to the front surface 128A of the wall 128.

A second portion 134B of the first seal membrane 134 is parallel to the longitudinal axis A102 and is attached on a peripheral outer surface 132B of the skirt 132.

A third portion 134C of the first seal membrane 134 connects its first and second portions 134A and 134B.

When it is attached to the body 122, the first fluid tight membrane 134 closes the volume V4, in complement to the elastomeric stopper 44.

Advantageously the first membrane 134 is a rectangular band, with its first portion 134A located at one end, its second portion 134B located at the opposite end and its third portion 134C located between its first and second portions.

The seal membrane 134 may include adhesive on one side and, in order to adhere to the cap 102, it only requires putting its portions against the respective parts of the body 122 i.e. the first portion 134A against the front surface 128A and the second portion 134B against the peripheral outer surface 132B. Preferably, the adhesive is disposed only on the first portion 134A and 134B and not disposed on the third portion 134C.

During manufacturing of the cap 102, the seal membrane 134 can be prepared by applying, on an active surface of the first portion 134A, which is configured to lay against the wall 128 in the configuration of figure 1, a first layer on a first adhesive compatible with the removal of the membrane 134 with respect to the wall 128.

As an example, this first adhesive can be a water based adhesive or a hot-melt adhesive.

An active surface of the second portion 134B, which is configured to lay against the external surface 132B, can be covered with a second layer of a second adhesive having stronger bonding properties than the bonding properties of the first adhesive. In one preferred embodiment, the second portion 134B of the seal membrane 134 is not supposed to be removed from the body 122.

As an example, the second adhesive can be an acrylic glue.

Alternatively the first seal membrane 134 can be attached to the body 122 by the use of glue, welding or any other method known to the skilled person, allowing posterior removal of the seal membrane 134.

In case the first seal member is attached to the body 122 by welding, it is possible to apply heat for a longer time on the second portion 134B of the seal membrane 134 than on its respective first portion 134A. Thus, the attachment obtained by welding is less in the zone where the first portion 134A is attached than in the zone where the second portion is attached. It is also possible to use a higher temperature for heating in the zone where the second portion 134B is attached, and a lower temperature for heating in the zone, where the first portion is attached to the corresponding parts of the body 102.

Alternatively or cumulatively, one can also differentiate the level of thermal energy, by applying a lower level of thermal energy on the first portions 134A of the first seal membrane 134 and a higher level of thermal energy on the second portion 134B of the first seal membrane 134.

Alternatively, the respective portions of the first seal membrane 134 can be attached to the body 122 by mechanical engagement with reliefs borne by the body 122. These reliefs can be grooves, notches or ridges. The ridges can result from knurling. In such cases, a first roughness used for the relief cooperating with the first portion 134A of the first seal membrane 134 can be smaller than the second roughness of the reliefs used with the second portion 134B of these membranes. The smaller and larger roughness may refer to Ra according to the EN ISO 4287, Sa according to ISO 25178 or any other roughness parameter known to the skilled person.

The different alternatives mentioned here above, which induce differentiated attachments for the second portion 134B and the first portion 134A of the first seal membrane, can be combined. In other words, it is possible to use different types of adhesives, different amounts of adhesive, different time period for applying heat, different levels of thermal energy and/or different roughness in a combined manner to obtain a different attachment for the second portion 134B as compared to the attachment of the first portion 134A of the first seal membrane with the body 122.

Preferably, irrespective of the type of attachment of the first seal membrane 134 to the body 122, no adhesive is applied on the third portion 134C of the membrane 134.

In the configuration of figure 1, the first portion 134A of the first seal membrane 134 attached to the front surface 128A covers and seals the through hole 130, so that the front surface 44A of the septum 44 is completely isolated from the outside by the closing wall 128 equipped with the first portion 134A of the first membrane 134.

In the configuration of figure 1, the third portion 134C of the seal membrane 134 extends in a plane, which is oblique with respect to the longitudinal axis A102. In particular, the direction of the third portion 134C is not parallel to the longitudinal axis A102.

The connection system 100 also includes a needle module 104 having a body 142 equipped with a hollow needle 144. Preferably, the body 142 is made of an integral piece of synthetic material, preferably a material that can be molded, for example acrylonitrile butadiene styrene, methylmethacrylate acrylonitrile butadiene styrene or MABS, polyamide, polybutylene terephthalate, polycarbonate, polyoxymethylene (acetal), polypropylene or polytetrafluoroethylene.

The body 142 comprises a protrusion 148 configured for supporting the needle 144 in a position aligned with a longitudinal axis A104 of the needle module 104, with a tip 144A of the needle 144 oriented towards the front of the needle module 104, that is opposite to the receiving part 6.

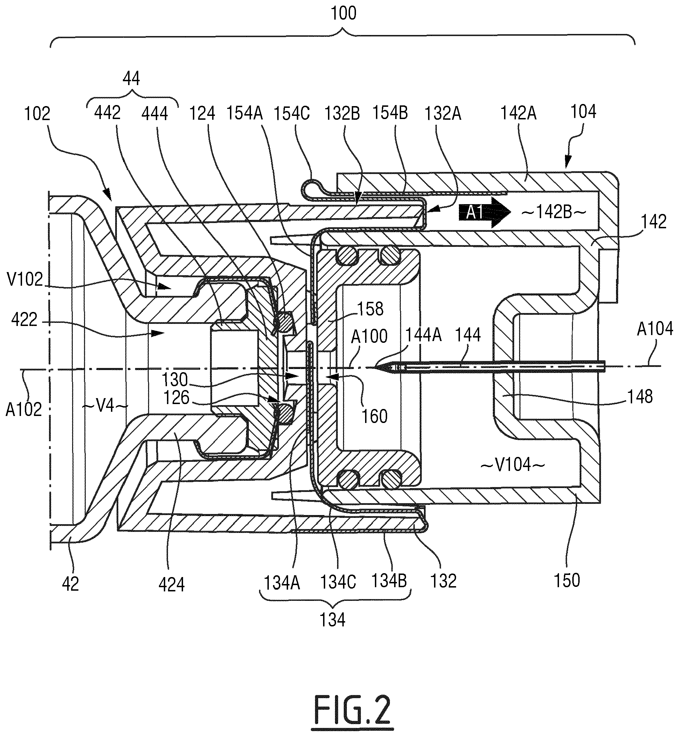

The needle module 104 also comprises a closure member 146 supported by the body 142. The closure member 146 is located on a front side of the needle module directed toward the container 4 during connection of these two elements, in particular in the configuration of figure 2.

The body 142 comprises a skirt 150 centered on the longitudinal axis A104.

A100 denotes a longitudinal axis of the connection system 100.

150A denotes a front edge of the skirt 150, oriented in the same direction as the tip 144A, that is toward the front of the needle module 104. This front edge 150A is a protruding relief of the needle module, oriented to the front of the needle module.

V104 denotes the internal volume of the needle module, defined by the skirt 150.

A lateral extension 142A is attached to the body 142 outside the internal volume V104. Preferably, the lateral extension 142A forms an integral part of the body. The lateral extension 142A is substantially parallel to the longitudinal axis A104 and is separated from the skirt 150 of the body 142 by a recess 146B.

The closure member 146 is slidably mounted within the skirt 150, so that it can be translated within the internal volume V104 of the needle module 104, along the longitudinal axis A104.

146A denotes a front surface of the closure member 146, which is orthogonal to the longitudinal axis A104 and oriented away from the receiving part 6.

The closure member 146 includes a front wall 158, which defines its front surface 146A and which is perpendicular to the longitudinal axis A104, and a skirt 159 provided with two grooves 153 on its outer surface.

The closure member 146 is equipped with two sealing rings 152 disposed in two grooves 153 located on the outer surface of the skirt 159. The rings 152 form sealing elements between the closure member 146 and the skirt 150. Thus, the closing member 146 is tightly mounted within the skirt 150. In principle another number of sealing rings 152 may be used, including, particularly a single sealing ring. At least one sealing ring can be made of a material providing liquid and gas tightness, for example synthetic rubber, polyurethane or polytetrafluoroethylene

Alternatively, other types of sealing elements known for the skilled person can be used, instead of the sealing rings 152, for insuring a tightness between the closing element 146 and the skirt 150, e.g. the internal surface of the skirt 150 can be covered by a layer of a synthethic rubber, polyutherane or polytetrafluoroethylene or other materials providing liquid and gas tightness. According to another alternative, the closure member 146 may be made from any of synthethic rubber, polyutherane or polytetrafluoroethylene or other material providing liquid and gas tighteness

A through hole 160 is arranged in the front wall 158. It is aligned on the longitudinal axis A104. The radial dimensions of the through hole 160 are large enough for the needle to pass through this hole without contact with the wall 158.

The needle module 104 comprises a second seal membrane 154 attached to the body 142 and to the closure member 146.

In the example, this second membrane 154 can be made of any suitable material impermeable for the liquid and for bacteria, but permeable to air. The particular examples of such materials are non-woven fabrics, such as the non-woven fabric made of high density polyethylene fibers known with a brand name Tyvek^{®} or the non-woven fabrice made of high density polypropylene fibers known with a brand name PelTek. A porous paper may be used as well as such membrane. Use of a sterile membrane permeable to air is particularly advantageous as it allows to conduct steam sterilization which is commonly used for the drugs which usually cannot be handled with gamma sterilization.

This seal membrane 154 is preferably made of the same material as the first membrane 134.

In another embodiment, the membranes 134 and 154 are made of two different materials or more.

A first portion 154A of the second seal membrane 154 is perpendicular to the longitudinal axis A104 and attached to the closure member 146, more particularly to its front surface 146A.

A second portion 154B of the second seal membrane 154 is parallel to the longitudinal axis A104 and attached to the lateral extension 142A of the body 142, on a side of this extension oriented toward the longitudinal axis A104 and the skirt 150.

A third portion 154C of the second seal membrane 154 connects its first and second portions 154A and 154B.

When it is attached to the closure member 146 of the needle module 104, the second seal membrane 154 closes the volumes V6 and V104, thus in particular the volume V6.

Advantageously, the second seal membrane 154 is a rectangular band, with its first portion 154A located at one end, its second portion 154B located at the opposite end and its third portion 154C located between its first and second portions.

In the configuration of figure 1, the first portion 154A of the second seal membrane 154 covers and seals the through hole 160, so that the inner volume V104 of the needle module 104 is completely isolated from the outside by the closure member 146 equipped with the first portion 154A of the second seal membrane 154 and with the sealing rings 152. Thus, in the configuration of figure 1 which is prior to the opening the connection, the needle tip 144A is located within the internal volume V104, enclosed within the body 142, and protected from external contamination.

The third portion 154C of the second seal membrane 154 extends in a direction non-parallel to the main axis A100. Advantageously, the third portion 154C of the second seal membrane 154 extends globally in a plane perpendicular to the longitudinal axis A104 and includes a curve for connection to the second portion 154B.

The attachment of the second portion 154B to the body 142 is stronger than the attachment of the first portion 154A to the front face 146A of the closure member 146. This difference in attachment, preferably can be obtained in the same ways as described here above for the first seal membrane 134.

To establish a connection between the internal volume V4 of the drug container 4 and the internal volume V6 of the receiving part 6, the drug container 4 equipped with the cap 102 and the receiving part 6 equipped with the needle module 104 are substantially aligned with each other on the longitudinal axis A100 of the connection system 100, with their respective front sides facing each other. The longitudinal axes A102 and A104 coincide with each other on the main axis A100 and the front surface 128A of the wall 128 faces the needle module 104, whereas the front surface 146A of the closure member 146 faces the cap 102.

This corresponds to a first step of a method for opening a connection between items 4 and 6, which leads to the configuration of figure 1

In a second step, the cap 102 and the needle module 104 are moved towards each other along the main axis A100, which leads to the configuration of figure 2. This relative translational movement between parts 102 and 104 may be realized by axial displacement of one of the cap 102 and the needle module 104 towards the other element or by simultaneous displacement of the cap 102 and the needle module 104 towards each other.

In this configuration, the front edge 132A of the skirt 132 has pushed the third portion 154C of the second seal membrane 154 within the recess 142B defined by the body 142, between the lateral extension 142A and the skirt 150.

Since the attachment between the second portion 154B of the second seal membrane 154 and the lateral extension 142A of the main body is stronger than the attachment between its first portion 154A and the closure member 146, the movement of the third portion 154C into the recess 142B induces that the first portion 154A is progressively removed from the front surface 146A of the closure member 146, whereas the second portion 154B stays attached to the lateral extension 142A. The first portion 154A of the of the second seal membrane 154 is displaced by the front edge 132A with respect to the receiving part 6, in particular in a direction away from the through hole 160.

This results in the through hole 160 becoming opened, since it is no more covered by the first portion 154A of the second seal membrane 154. The through hole 160 is ready to be passed through by the needle 144.

In the position of figure 2, the front edge 150A of the skirt 150 is in contact with the third portion 134C of the first seal membrane 134, but the two portions 134A and 134B of the first seal membrane 134 remain at least partially attached to the cap 102.

In the configuration of figure 2, the third portion 154C of the second seal membrane 154 has already been pushed in a direction away of the drug container 4, so that its first portion 154A is removed from the front surface 146A of the closure member 146, whereas the front edge 150A of the skirt 150 has not yet pushed the third portion 134C of the first seal membrane 134 enough to remove its first portion 134A from the wall 128.

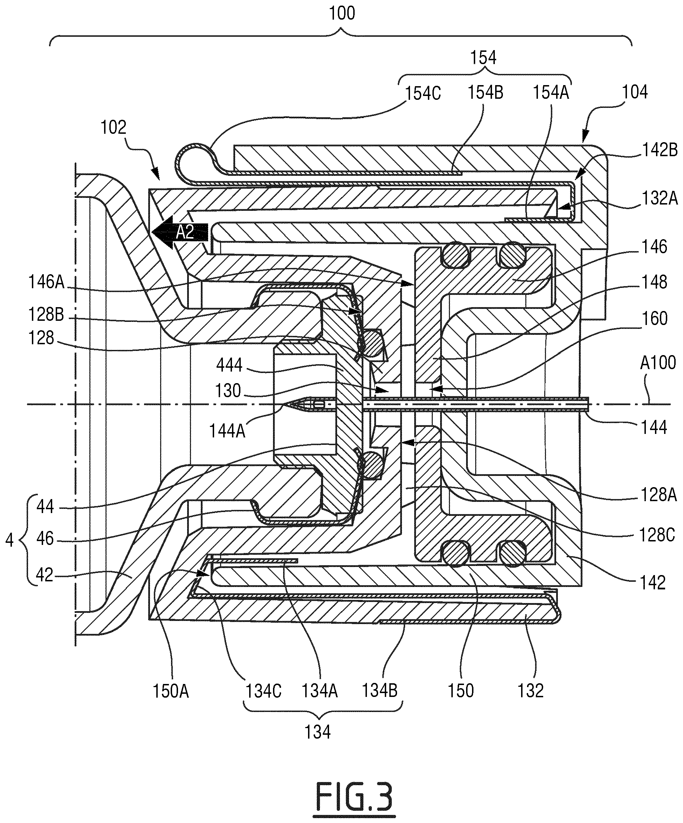

By continuing the connection movement between the cap 102 and the needle module 104 along the main axis A100, one reaches the configuration of figure 3, where the wall 128, the septum 44 and the neck 424 are engaged in the internal volume V104 of the needle module 104. This is possible since the closure member 146 has been translated within this volume, in the direction of arrow A1 on figure 2, towards the external surface of the recess 148.

During the transition between the configuration of figure 2 and the configuration of figure 3, the front edge 150A of the skirt 150 exerts on the third portion 134C of the first seal membrane 134 a pushing force in a direction away from the needle 144, represented by arrow A2 on figure 3, which results in removing the first portion 134A from the wall 128. In other words, the first portion 134A of the first membrane 134 is displaced by the front edge 150A with respect to the drug container 4, in particular in a direction away from the septum 44. This displacement gives access to the through hole 130 which can be passed through by the needle 144 to pierce the central area 444 of the septum 44. This occurs since the third portion 134C of the first seal membrane 134 extends in a direction non-parallel to the main axis A100, on the path of the front edge 150A moving toward the rear side of the cap 102, which is opposite to its front side.

The removal of the first portion 134A of the first seal membrane 134 from the wall 128 results from the fact that the attachment of the second portion 134B of the first seal membrane 134 on the external surface 132B of the external skirt 132 is stronger than the attachment of the first portion 134A of the first seal membrane 134 on the wall 128.

In the configuration of figure 3, the front surface 146A of the closure member 146 lies against the circular rib 128C.

Thus, an exclusively translational relative movement of the two parts 102 and 104 of the connecting system 100 of the invention towards each other, along the main axis A100 and in the directions of the arrows A1 and A2, results in the two protective seal membranes 134 and 154 being automatically removed, for example peeled-off, in the zones where they cover the through holes 130 and 160, without any further action of a user. This translational relative movement is also a translational relative movement between the two elements 4 and 6.

Therefore, opening of a connection between the container 4 and the receiving part 6 is particularly simple and easy to implement.

In particular, this simple opening of the connection can be implemented in a non-sterile environment without compromising the sterility of the elements being connected.

In the preferred embodiment, some engagement means may be provided on the bodies 122 and 142 to immobilize the needle module 104 with respect to the cap 102. Preferably, these engagement means are made of snap members, for example hooks or latch elements.

As visible in Fig. 4 the skirt 132 is divided into several longitudinal portions 137 separated by one or several slots 133. Some of the longitudinal portions 137 are provided with first snap members 123. The lateral extension 142A is equipped with four lateral protrusions 143, which extend laterally from a stem 142C of the lateral extension 142A and form second snap members.

When the cap 102 and the needle module 104 are translated from the configuration of figure 1 to the configuration of figure 3, via the configuration of figure 2, the first snap members 123 engage into the recess and become hooked beyond the second snap members, as shown by the comparison of inserts A), B) and C) of figure 4.

The geometry of the snap members 123 and 143 is not limitative. Other geometries can be selected, according to the needs. Moreover, the second snap members 143 can be located on the body 142 of the needle module 104 elsewhere than on the lateral extension 142A.

In addition, the skirt 150 of the needle module 104 is provided with external reliefs, in the form of a rib 153, which follow the shape of a rectangle on the outer surface of the skirt 150. When the cap 102 and the needle module 104 are connected, the ribs 153 progressively enter the slots 133 providing better alignment between the cap 102 and the needle module 104 during a relative translational movement.

In another embodiment, a part of the third portion 134C of the first seal membrane 134 and/or a part of the third portion 154C of the second seal membrane 154 extends in a direction parallel to the main axis A100, this part being not located on the path of the protruding parts made by the edges 132A and 150A.

In a not shown embodiment of the invention, the closing of the volume V104 of the needle module 104 is made by another part supported by the body, instead of the slidable closure member 146. This part can be integral with, or fixedly mounted on, the body 142. This part may be a diaphragm, bent, pierced or torn off by the wall 128 penetrating within the volume V104 or by the needle 144. If this part is a diaphragm to be torn off by the wall 128 or the needle 144, it includes a central portion aligned with the needle along the main axis A100 and covered by the first portion 154A of the second membrane 154.

In an alternative embodiments, the connection system 100 does not comprise the needle or nor the septum to be pierced. The connection system may include a module other than the needle module with the closure element integral with the body or fixed to the body of said module.

In a not shown embodiment of the invention, the first and second elements 4 and 6 are parts of a mixing system or reconstitution system, in particular for a drug.

As long as they are compatible, the embodiments and alternatives mentioned here above may be combined.

## Claims

1. A connection system (100) for opening a connection between a first internal volume (V4) of a first element (4), the first volume being closed by a first seal membrane (134), and a second internal volume (V6) of a second element (6), the second volume being closed by a second seal membrane (154), wherein the connection occurs via a relative translational movement (A1, A2) between the first and second elements (4, 6) along a main axis (A100) of the connection system, **characterized in that** the relative translational movement (A1, A2) between the first and second elements (4, 6) induces a displacement of at least a first portion (134A) of the first membrane (134) with respect to the first element (4) and a displacement of at least a first portion (154A) of the second membrane (154) with respect to the second element (6).

2. The connection system of claim 1, **characterized in that** the first element (4) and the second element (6) comprise engagement members (123, 143), preferably in the form of snap members.

3. The connection system of any of the preceding claims, **characterized in that** one of the first element (4) and second element (6) comprises a through hole (130) covered by a perforable element (444) and a seal membrane (134) and the other one of the first element (4) and second element (6) comprises a piercing member (144) configured to pierce the perforable element (444) to open the connection.

4. The connection system of a claim 3, **characterized in that** the other one of the first element (4) and the second element (6), which comprises the piercing member (144), further comprises a slidable closure member (146) located on a side of the other one of the first element (4) and the second element (6) directed toward the one of the first element (4) and second element (6), wherein the slidable closure (146) further comprises a through hole (160) aligned with a tip (144A) of the piercing member (144).

5. The connection system of a claim 4, **characterized in that** the slidable closure member (146) is equipped with at least one sealing member (152) configured for making a tight connection between the slidable closure member (146) and a body (142) of the other one of the first element (4) and the second element (6).

6. The connection system of any of claims 4 and 5, **characterized in that** one of the first seal membrane (134) and the second seal membrane (154) is attached to the slidable closure member (146).

7. The connection system of any of the preceding claims, **characterized in that** the first element (4) and the second element (6) comprise protruding reliefs (132A, 150A) configured to push parts (134C, 154C) of the seal membranes (134, 154) disposed on the other of the first element (4) and the second element (6) and causing removing of the seal membranes and opening the connection.

8. The connection system of any preceding claim, **characterized in that** the first seal membrane (134) includes:
- a first portion (134A) which is substantially perpendicular to the main axis (A100) and attached to the first element (4),
- a second portion (134B) substantially parallel to the main axis and attached to the first element (4), and
- a third portion (134C) connecting the first portion to the second portion;
and the second seal membrane (154) includes:
- a first portion (154A) which is substantially perpendicular to the main axis (A100) and attached to the second element (6),
- second portion (154B) substantially parallel to the main axis and attached to the second element (6), and
- a third portion (154C) connecting the first portion to the second portion;
wherein before connection, the third portions (134C, 154C) of the first and second seal membranes (134, 154) extend at least partially in a direction non parallel to the main axis (A100) and
wherein the first seal membrane (134) and the second seal membrane (154) are configured so that during opening the connection between the first element (4) and the second element (6), said first portions (134A, 154A) detach from the first element (4) while said second portions (134B, 154B) remains attached to the first element (4) and second element (6).

9. The connection system according to any of the preceding claims, **characterized in that** the first element (4) and the second element (6) are parts of a drug delivery system.

10. The connection system according to claim 9, **characterized in that** the drug delivery system includes a disposable part comprising a drug container (4) and a reusable part comprising a mechanism for delivering the drug, wherein the connection system allows to open a fluid path between the drug container (4) and another element of the disposable part.

11. The connection system according to any of claims 1 to 8, **characterized in that** first element (4) and the second elements (6) are parts of a mixing system or reconstitution system.

12. A manufacturing process for manufacturing the connection system of any of preceding claims, comprising at least the following steps:
a. providing the first (4) and the second element (6) having the first volume (V4) and the second volume (V6), respectively, wherein the first volume (V4) and the second volume (V6) are open,
b. providing a first seal membrane (134) and a second seal membrane (154)
c. attaching the first seal membrane (134) to the first element (4) and the second seal membrane (154) to the second element (6) causing closing of the first volume (V4) and the second volume (V6), respectively,
**characterized in that** the attachment of the first seal membrane (134) and the attachment of the second seal membrane (154) are configured so that said membranes can be displaced in result of the relative translational movement between the first element (4) and the second element (6), causing opening of the connection.

13. The manufacturing process according to claim 12, **characterized in that** the first seal membrane (134) includes:
- a first portion (134A) substantially perpendicular to the main axis (A100) and attached to the first element (4),
- a second portion (134B) substantially parallel to the main axis and attached to the first element (4), and
- a third portion (134C) connecting the first portion to the second portion;
and the second seal membrane (154) includes:
- a first portion (154A) substantially perpendicular to the main axis (A100) and attached to the second element (6),
- a second portion (154B) substantially parallel to the main axis and attached to the second element (6), and
- a third portion (154C) connecting the first portion to the second portion,
wherein at least the first portion (134A) of the first seal membrane and the second portion (134B) of the first seal membrane are attached to the first element and at least the first portion (154A) of the second seal membrane and the second portion (154B) of the second seal membrane are attached to the second element,
wherein attachment of the first seal membrane (134) and the second seal membrane (154) is configured so that during opening the connecting between the first element (4) and the second element (6), said first portions (134A, 154A) detach from the first element (4) and the second element (6) before said second portions (134B, 154B) detach from the first element (4) and second element (6).

14. The manufacturing process according to any of claims 12 and 13, **characterized in that** attaching the first seal membrane (134) to the first element (4) and the second seal membrane (154) to the second element (6) is made with the use of adhesive, preferably adhesive supported by the first seal membrane and the second seal membrane, or welding.

15. A method for opening a connection between a first element (4) and a second element (6) using the connection system according to any of claims 1-11, wherein the method includes at least the following steps:
d. substantially aligning the first element (4) and the second element (6) along a main axis (A100) of the connection system (100),
e. relative translational movement between the first element (4) and the second element (6), along the main axis,
f. displacement (A2) of at least a part of the first seal membrane (134) with respect to the first element (4) and displacement (A1) of at least a part of the second seal membrane (154) with respect to the second element (6),
g. opening the connection between the first internal volume (V4) of the first element (4) and the second internal volume (V6) of the second element (6),
and wherein step f. is a result of step e. and step g. is a result of step f.

16. The method according to claim 15, **characterized in that** the first element (4) and the second element (6) comprise protruding reliefs (132A, 150A) and that the step f. is a result of pushing the first seal membrane (134) with the protruding relief (150A) of the second element and pushing the second seal membrane (154) with the protruding relief (132A) of the first element.

17. The method according to any of claims 15 and 16, **characterized in that** one of the first element (4) and the second element (6) comprises a perforable element (444) and the other one of the first element (4) and second element (6) comprises a piercing member (144) configured to pierce the perforable element after step g., wherein revealing a tip (144A) of the piercing member is a result of a relative translational movement between the first and second elements (4, 6).
